# EUROPÄISCHE PATENTSCHRIFT

(11) **EP 2 445 461 B1**
(45) Veröffentlichungstag und Bekanntmachung des Hinweises auf die Patenterteilung: **11.06.2014**
(21) Anmeldenummer: 09768502.8
(22) Anmeldetag: 07.12.2009
(51) Int. Cl.: A61F 9/008, A61F 9/009

(54) **VORRICHTUNG FÜR DIE OPHTHALMOLOGISCHE LASERCHIRURGIE**
DEVICE FOR OPHTHALMIC LASER SURGERY
DISPOSITIF POUR CHIRURGIE LASER OPHTALMOLOGIQUE

(43) Veröffentlichungstag der Anmeldung: 02.05.2012
(73) Patentinhaber: WaveLight GmbH, 91058 Erlangen (DE)
(72) Erfinder: DONITZKY, Christof, 90542 Eckental (DE); VOGLER, Klaus, 90542 Eckental (DE); KITTELMANN, Olaf, 14163 Berlin (DE); GORSCHBOTH, Claudia, 90411 Nürnberg (DE)
(74) Vertreter: Frenkel, Matthias Alexander
(86) Internationale Anmeldenummer: PCT/EP2009/008747
(87) Internationale Veröffentlichungsnummer: WO 2011/069516

(56) Entgegenhaltungen:
- WO-A1-2011/015205
- US-A1- 2003 153 904
- US-A1- 2007 135 805
- US-A1- 2008 039 769
- US-A1- 2009 069 798

## Beschreibung

Die Erfindung betrifft eine Vorrichtung für die ophthalmologische Laserchirurgie sowie ein zugehöriges Verfahren.

Gepulste Laserstrahlung kommt bei zahlreichen Techniken der Behandlung des menschlichen Auges zum Einsatz. Die örtliche Steuerung des Strahlfokus des Laserstrahls in z-Richtung (gemeint ist hiermit gemäß üblicher Notation die Ausbreitungsrichtung des Laserstrahls) erfolgt stets mit Bezug auf einen bekannten Referenzpunkt oder eine bekannte Referenzfläche im Koordinatensystem der Laservorrichtung.

Je nach Behandlungsart können unterschiedliche Referenzpunkte oder Referenzflächen als Bezug für die z-Steuerung des Strahlfokus dienen. Bei einigen dieser Techniken wird das zu behandelnde Auge gegen ein transparentes Kontaktelement gedrückt, das mit seiner augenzugewandten Kontaktfläche eine Referenzfläche für die Positionierung des Strahlfokus in z-Richtung bildet. Insbesondere Behandlungstechniken, die der Erzeugung von Schnitten (Inzisionen) im Augengewebe mittels fokussierter Femtosekunden-Laserstrahlung dienen, bedienen sich häufig solcher Kontaktelemente als z-Referenz für den Laserfokus. Indem das Kontaktelement so gegen das Auge gedrückt wird, dass sich eine anschmiegende flächige Anlage des Auges an der augenzugewandten Kontaktfläche des Kontaktelements einstellt, gibt das Kontaktelement die z-Lage der Augenvorderfläche vor. Durch Referenzierung des Strahlfokus in z-Richtung gegenüber dieser Kontaktfläche des Kontaktelements ist dann sichergestellt, dass der Schnitt bzw. die einzelne Photodisruption (die Schnitterzeugung im menschlichen Auge mittels gepulster Femtosekunden-Laserstrahlung beruht regelmäßig auf dem Effekt des sogenannten laserinduzierten optischen Durchbruchs, der zu einer Photodisruption führt) an der gewünschten Position in der Tiefe des Augengewebes liegt.

Lasertechnisch erzeugte Schnitte kommen beispielsweise bei der sogenannten Fs-LASIK vor, bei der ein in der Fachwelt als Flap bezeichnetes anteriores Deckelscheibchen der Kornea mittels Femtosekunden-Laserstrahlung freigeschnitten wird, um anschließend wie bei der klassischen LASIK-Technik (LASIK: Laser In Sito Keratomileusis) den noch in einem Scharnierbereich (*hinge*) am restlichen Korneagewebe hängenden Flap zur Seite zu klappen und das so freigelegte Gewebe mittels UV-Laserstrahlung ablatierend zu bearbeiten. Ein anderer Anwendungsfall für die Anbringung intrageweblicher Schnitte im Augengewebe ist die sogenannte korneale Lentitelextraktion, bei der innerhalb des Korneagewebes ein linsenförmiges Scheibchen mittels Femtosekunden-Laserstrahlung rundum herausgeschnitten wird. Dieses Scheibchen wird anschließend durch einen zur Augenoberfläche herausgeführten zusätzlichen Schnitt entnommen (der zusätzliche Schnitt wird entweder mittels eines Skalpells oder ebenfalls mittels Femtosekunden-Laserstrahlung erzeugt). Auch bei Hornhauttransplantationen (Keratoplastik) oder für andere Inzissionen z.B. für korneale Ringsegmente kann die Schnitterzeugung in der Kornea mittels fokussierter gepulster Laserstrahlung durchgeführt werden.

Aus Hygienegründen ist das die Kontaktfläche tragende Kontaktelement (Applikator) oftmals ein Wegwerfartikel, der vor jeder Behandlung auszutauschen ist. Gewisse Fertigungstoleranzen sind bei der Herstellung der Kontaktelemente auch bei größter Fertigungspräzision regelmäßig nicht auszuschließen. Daher kann nach einem Austausch des Kontaktelements die z-Lage der augenzugewandten Kontaktfläche - wenn auch nur geringfügig - anders sein als bei dem zuvor benutzten Kontaktelement. Bei Laserbehandlungen mittels fokussierter Femtosekunden-Laserstrahlung werden möglichst kleine Fokusdurchmesser angestrebt, um die photodisruptive Wirkung lokal möglichst eng zu begrenzen. Moderne Geräte arbeiten beispielsweise mit Fokusdurchmessern im niedrigen einstelligen µm-Bereich. Oftmals ist bei der Durchführung von Eingriffen mittels Femtosekunden-Systemen die Schnitttiefe im Zielgewebe mit einer extrem hohen Genauigkeit (Schnitttiefentoleranzen < 5 µm) zu definieren. Wie zuvor beschrieben, sind in der Regel bei solchen Eingriffen das zu behandelnde Gewebe und das optische System des Lasers durch ein Kontaktelement fest aneinander gekoppelt, um die erforderliche Schnitttiefe bei entsprechender Präzision in z-Richtung zu erreichen. Dies verlangt eine entsprechend hohe Fertigungsgenauigkeit des Kontaktelements, die jedoch nicht immer gewährleistet werden kann. Bei verminderter Fertigungspräzision des Kontaktelements stellt sich deshalb das Problem einer in z-Richtung unpräzisen Schnittführung im Korneagewebe, d.h. die Fertigungstoleranzen für diese Kontaktelemente gehen direkt in die Ungenauigkeiten für die Schnitttiefe im Gewebe ein.

Im Stand der Technik werden im allgemeinen Applikatoren verwender die mit entsprechendem Aufwand präzise gefertigt wurden. Bei der Installation dieser Applikatoren wird das optische System des Lasers anhand eines Referenzapplikators unter Ausnutzung der Wechselwirkung zwischen Laserstrahlung und Material auf die geforderte Distanz zwischen dem optischen System und der Schnittebene eingestellt. Dies ist beispielsweise bereits aus der WO 2004/032810 bekannt.

Der Abstand zwischen Gewebe und Lasersystem und damit direkt die reale Schnitttiefe im Gewebe wird wesentlich durch die Dimension des Applikators, d.h. die reale optische Länge des Applikators in z-Richtung, bestimmt. Dies macht es zum Erreichen der erforderlichen Präzision der Schnitttiefe notwendig, dass die Applikatoren mit entsprechend kleinen Toleranzen bezüglich ihrer Dimension gefertigt werden müssen (die relative Längengenauigkeit liegt bei deutlich << 1‰), was die Herstellungskosten dieser Applikatoren deutlich erhöht und sich insbesondere bei in großen Stückzahlen erforderlichen Einmalartikeln direkt auf die Behandlungskosten und damit auf die sogenannten "Costs of Ownership" auswirkt.

Aus der PCT/EP2009/006879 der Anmelderin ist bekannt, Fertigungsungenauigkeiten des Kontaktelements zu berücksichtigen und auszugleichen. Hierfür wird mittels einer Messeinrichtung eine Positionsvermessung der Kontaktfläche bezogen auf die Ausbreitungsrichtung des Behandlungslaserstrahls durchgeführt und mittels einer mit der Messeinrichtung verbundenen elektronischen Auswerte- und Steueranordnung der Fokusort des Behandlungslaserstrahls abhängig von den durch die Messeinrichtung gewonnenen Positionsmessdaten eingestellt.

Die Druckschriften US 2008/0039769 A1 und US 2007/0135805 A1 offenbaren Implantat-Rohling, der zur Verwendung für die Veränderung der Krümmung der Hornhaut eines Patienten geeignet ist, umfassend: eine erste Oberfläche, die zum direkten Platzieren auf die Oberfläche der Lebendhornhaut des Patienten ausgebildet ist; eine zweite. Oberfläche, die ausgebildet ist, um einem Laserstrahl ausgesetzt zu werden; wobei der Implantat-Rohling ein Material umfasst, dessen Eigenschaften es Licht mit einer Wellenlänge innerhalb des sichtbaren Spektrums ermöglicht, durch dieses hindurch zu treten und im Wesentlichen das gesamte Licht mit einer Wellenlänge innerhalb des Laserlichtspektrums davon abhält, durch dieses hindurch zu treten, wobei der Implantat-Rohling weiterhin eine Wandoberfläche umfasst, die sich zwischen der ersten Oberfläche und der zweiten Oberfläche erstreckt, und eine Öffnung in dem Implantat-Rohling definiert, und dass dieser Implantat-Rohling eines aus dem Folgenden umfasst: (i) einen Bereich, der sich von der Öffnung zu einem Randbereich des Implantat-Rohlings erstreckt und eine Dicke aufweist, die größer als die Dicke eines angrenzenden Abschnitts des Implantat-Rohlings ist oder (ii) einen Bereich, der sich von der Öffnung zu einem Randbereich des Implantat-Rohlings erstreckt und eine Dicke aufweist, die kleiner als die Dicke eines angrenzenden Abschnitts des Implantat-Rohlings ist.

Die Druckschrift US 2009/0069798 A1 offenbart ein System zum Anwenden einer Therapie bei einem Auge, die selektiv ein Kühlmittel auf die korneale Oberfläche aufbringt, um einen wärmebedingten Schaden an der kornealen Oberfläche zu verhindern.

Die Druckschrift WO2011/015205 (Stand der Technik nach Artikel 54(3) EPÜ) offenbart eine Einrichtung für die laserchirurgische Ophthalmologie, umfassend eine Quelle gepulster Femtosekunden-Laserstrahlung, Komponenten zur Führung und Fokussierung der Laserstrahlung auf oder in ein Gewebe eines zu behandelnden Auges, eine die Quelle steuernde Steuereinheit, welche dazu eingerichtet ist, die Quelle zwischen mindestens zwei Betriebsmodi mit jeweils unterschiedlichen Strahlungseigenschaften der Laserstrahlung umzusteuern, wobei in einem ersten Betriebsmodus die Strahlungseigenschaften der Laserstrahlung auf die Anbringung einer Inzision in dem Gewebe abgestimmt sind und in einem zweiten Betriebsmodus die Strahlungseigenschaften der Laserstrahlung auf eine Gewebeverschweißung bzw. -vernetzung abgestimmt sind.

Die aus dem Stand der Technik bekannten Vorgehensweisen berücksichtigen zwar Fertigungsungenauigkeiten des Kontaktelements oder versuchen diese durch möglichst hohe Präzision (bei gleichzeitig hohen Kosten) zu vermeiden, lassen aber weitere die Einstellungsgenauigkeit des Fokuspunkts in z-Richtung beeinflussende Faktoren außer Acht.

Die effektive Schnitttiefe ist zusätzlich zu den genannten Fertigungstoleranzen von Temperaturdriften der Dimension des Applikators sowie der effektiven Brennweite der gesamten Optik abhängig, d.h. die reale optische Länge des Applikators in Ausbreitungsrichtung des Behandlungslaserstrahls sowie die Brennweite der Optik eines Lasersystems variieren abhängig von dem Temperatureinsatzbereich. Die genannten Drifte können sich im konventionellen Temperatureinsatzbereich medizinischer Geräte von 15°C - 35°C leicht auf 30 µm bis zu 50 µm aufsummieren. Damit sind die angestrebten Schnitttiefentoleranzen < 5 µm schwer oder nicht mehr erreichbar.

Es ist eine Aufgabe der vorliegenden Erfindung, eine Vorrichtung für die ophthalmologische Laserchirurgie sowie ein zugehöriges Verfahren bereitzustellen, welche eine präzisere Laserbehandlung eines Auges ermöglichen.

Gemäß der Erfindung werden eine Vorrichtung und ein Verfahren gemäß den unabhängigen Ansprüchen bereitgestellt. Entwicklungen sind in den abhängigen Ansprüchen dargestellt.

Es ist eine Vorrichtung für die ophthalmologische Laserchirurgie vorgesehen, die folgende Komponenten umfasst: ein optisches Abbildungssystem zum Abbilden eines Behandlungslaserstrahls auf einen Fokuspunkt, eine Temperaturmesseinrichtung zur Messung einer dem Abbildungssystem zugeordneten Temperatur und eine mit der Temperaturmesseinrichtung verbundene elektronische Steueranordnung, welche dazu eingerichtet ist, die Fokuspunkteinstellung abhängig von der gemessenen Temperatur zu steuern.

Dabei umfasst die Vorrichtung vorzugsweise eine Kontaktfläche zur formenden Anlage eines zu behandelnden Auges sowie eine Strahlungsquelle zur Bereitstellung des Behandlungslaserstrahls. Weiter kann das Abbildungssystem vorzugsweise optische Komponenten zum Richten des Behandlungslaserstrahls durch die Kontaktfläche hindurch auf das Auge aufweisen.

Es wird bereitgestellt: eine Steuerung und/oder Nachregulierung einer beispielsweise voreingestellten Position des Laserstrahlfokus in z-Richtung (entsprechend der Ausbreitungsrichtung des Behandlungslaserstrahls) abhängig von der gemessenen Temperatur der kritischen, die Fokustiefe maßgeblich beeinflussenden Komponenten (z.B. des Objektivs, der Komponente zur Strahlaufweitung, etc.) und um die Vorrichtung. Die Voreinstellung des Fokuspunkts kann auf verschiedene Art und Weise erfolgen.

Die Position des Fokus in z-Richtung kann vorzugsweise dadurch voreingestellt werden, dass die z-Position der Kontaktfläche bezüglich eines gegebenen Referenzpunkts in einem festen Koordinatensystem der laserchirurgischen Vorrichtung bekannt ist. Vorzugsweise wird hierbei ein Patientenadapter (Applikator) verwendet, dessen reale optische Länge in Ausbreitungsrichtung des Behandlungslaserstrahls (z-Richtung) hochgenau hergestellt wurde, so dass der Fokuspunkt auf die bekannte Länge voreingestellt werden kann. Längenveränderungen des Applikators oder Veränderungen der effektiven Brennweite der in der Vorrichtung enthaltenen optischen Komponenten auf Grund sich um die Vorrichtung ändernder Temperatur, können von der Temperaturmesseinrichtung erkannt und von der Steueranordnung entsprechend berücksichtigt werden. Vorzugsweise wurde der effektive optische Abstand (die reale optische Länge) zwischen der dem Auge zugewandten Fläche des Applikators (der Kontaktfläche) und der dem Auge abgewandten Fläche (der den optischen Komponenten der Vorrichtung zugewandten Fläche) bereits außerhalb der Vorrichtung gemessen und beispielsweise über eine Kodierung dem zugehörigen Applikator aufgeprägt. Diese Kodierung kann dann vorzugsweise von der Vorrichtung, z.B. automatisch oder manuell, ausgelesen werden und an die Steueranordnung weitergebeben werden. Basierend auf dem ausgelesenen Wert, kann die Steueranordnung zunächst den Fokuspunkt voreinstellen.

Alternativ kann zur Voreinstellung des Fokuspunkts vorzugsweise die z-Position der Kontaktfläche mit Bezug auf den gegebenen Referenzpunkt gemessen werden. Hierfür weist die Vorrichtung bevorzugt eine Messeinrichtung zur Positionsvermessung der Kontaktfläche bezogen auf die Ausbreitungsrichtung des Behandlungslaserstrahls auf. Die Messeinrichtung umfasst hierfür vorzugsweise eine einen Messstrahl bereitstellende zweite Strahlungsquelle. Die optischen Komponenten sind dann vorzugsweise dazu ausgebildet und angeordnet, auch den Messstrahl durch die Kontaktfläche hindurch auf das Auge zu richten. Die Messeinrichtung kann vorzugsweise mittels des Messstrahls Positionsmessdaten bereitstellen, welche für die gemessene Position der Kontaktfläche an mindestens einer Stelle derselben repräsentativ sind und kann die ermittelten Positionsmessdaten an die Steueranordnung weitergeben. Die elektronische Steueranordnung stellt in Antwort darauf den Fokuspunkt abhängig von den Positionsmessdaten vor. Für unterschiedliche Kontaktelemente kann sich je nach Herstellungsgenauigkeit eine unterschiedliche z-Lage der Kontaktfläche in dem Koordinatensystem bzw. ein unterschiedlicher effektiver optischer Abstand ergeben. Durch Auswertung der durch die Messeinrichtung durchgeführten Messung der z-Lage der Kontaktfläche und/oder der realen optischen Länge des Applikators, kann zunächst der Fokuspunkt in z-Richtung vorzugsweise voreingestellt werden, so dass Herstellungsungenaulgkeiten verringert oder vermieden werden. Basierend auf der gemessenen Temperatur kann anschließend der voreingestellte Fokuspunkt angepasst oder nachreguliert werden.

Die Anpassung oder Nachregelulierung des voreingestellten Fokuspunkts kann vorzugsweise in vorbestimmten Zeitintervallen z.B. dadurch erfolgen, dass nach vorgegebenen Zeitspannen eine wiederholte Messung der Temperatur durch die Temperaturmesseinrichtung erfolgt. Beispielsweise kann eine Nachregulierung vorzugsweise dann erfolgen, wenn die gemessene Temperatur die zuvor gemessene Temperatur um einen vorgegebenen Grenzwert übersteigt. In einem solchen Fall wäre von einer erheblichen Temperaturdrift auszugehen, welche eine Nachregulierung des Fokuspunkts erforderlich macht. Eine Verringerung des vorgegebenen Grenzwerts ermöglicht eine genauere, jedoch aufwändigere Nachregulierung des Fokuspunkts. Vorzugsweise sind die vorbestimmten Zeitintervalle und der vorgegebene Grenzwert in einem mit der Steueranordnung verbundenen Speicher hinterlegt, so dass die Steueranordnung bei Bedarf diese Werte auslesen und die Temperaturmesseinrichtung sowie die Nachregulierung des Fokuspunkts entsprechend steuern kann. Vorzugsweise erfolgt eine erneute Temperaturmessung nur dann, wenn beispielsweise von einem Benutzer eine entsprechende Anweisung an die Temperaturmesseinrichtung oder damit verbundene Komponenten eingegeben wird.

Die Temperaturmesseinrichtung kann vorzugsweise einen oder mehrere Temperatursensoren umfassen, die an einer oder mehrerer der optischen Komponenten angeordnet und mit der elektronischen Steueranordnung verbunden sind. Die optischen Komponenten bilden bevorzugt zum einen eine Scaneinheit zum Ablenken des Behandlungslaserstrahls in einer zu dessen Strahlengang orthogonalen Ebene (x-y-Ebene) oder eine 3D-Scaneinheit zum dreidimensionalen Ablenken des Behandlungslaserstrahls sowie zum anderen eine Fokussieroptik zum Fokussieren des Behandlungslaserstrahls in den Laserstrahlfokus aus. In diesem Fall sind vorzugsweise jeweils zwei Temperatursensoren an der Scaneinheit und an der Fokussieroptik angeordnet. Es können jedoch jeweils auch ein oder mehr als zwei Temperatursensoren an der Scaneinheit und an der Fokussieroptik angeordnet sein.

Zur Anpassung des Fokuspunkts umfassen die optischen Komponenten vorzugsweise zumindest ein steuerbares optisches Element. Vorzugsweise ist das steuerbare optische Element durch eine in Ausbreitungsrichtung des Behandlungslaserstrahls positionsveränderbare Linse gebildet. Zur Steuerung der Linse kann die Steueranordnung vorzugsweise in Abhängigkeit der gemessenen Temperatur eine Stellgröße zur Nachregulierung des voreingestellten Fokuspunkts erzeugen. Die Linse ist vorzugsweise mechanisch längs des optischen Strahlengangs verfahrbar oder umpositionierbar. In diesem Fall ist die Steueranordnung bevorzugt dazu eingerichtet, zur Anpassung des Fokuspunkts die Position der positionsveränderbaren Linse um die ermittelte Steilgröße zu verändern.

Alternativ ist es bevorzugt, eine steuerbare Flüssiglinse variabler Brechkraft zu verwenden. Bei unveränderter z-Position und auch ansonsten unveränderter Einstellung des Fokussierobjektivs lässt sich durch Verfahren einer längsverstellbaren Linse oder durch Brechkraftvariation einer Flüssiglinse eine z-Verlagerung des Strahlfokus erreichen, um dadurch den Fokuspunkt an die geänderte Temperatur anzupassen. Es versteht sich, dass zur z-Verstellung des Strahlfokus auch andere Komponenten bevorzugt sind, wie etwa ein verformbarer Spiegel.

Die Steueranordnung kann vorzugsweise ferner eine Speichereinheit aufweisen oder mit einer solchen verbunden sein, in welcher die Abhängigkeit des Fokuspunkts von der Temperatur als eine Funktion gespeichert ist. Die Temperaturabhängigkeiten aller in der Vorrichtung verwendeten Materialien und aller in der Vorrichtung auftretenden Abstände (beispielsweise die Abstände der optischen Komponenten voneinander oder die reale optische Länge des Applikators) können vorzugsweise verwendet werden, um einen Temperaturgang der effektiven Brennweite der optischen Komponenten ausgehend von einer Referenztemperatur zu berechnen Der Temperaturgang wird vorzugsweise für die Scaneinheit und die Fokussieroptik getrennt ermittelt und aufgezeichnet, kann jedoch auch für diese gemeinsam berechnet werden. Der ermittelte Temperaturgang kann vorzugsweise als Kurvenschar in der Speichereinheit hinterlegt und bei Bedarf von der Steueranordnung abgefragt und dazu verwendet werden, den Fokuspunkt basierend auf der gespeicherten Kurvenschar anzupassen.

Durch die Berücksichtigung der Temperatur bei der Nachregulierung des Fokuspunkts des Behandlungslaserstrahls durch die Steueranordnung, werden auf Grund von Temperaturschwankungen auftretende Veränderungen der effektiven Brennweite sowie des effektiven optischen Abstands des Applikators ausgeglichen. Dadurch wird erreicht, dass ein im Auge zu realisierendes Schnittmuster bzw. ein zu realisierendes Muster von Photodisruptionen tatsächlich an der gewünschten Stelle in der Tiefe des Auges (also an der gewünschten Stelle in z-Richtung) liegt. Auf diese Weise sind hochpräzise Schnitttiefen beispielsweise bei der Herstellung eines LASIK-Flaps, bei kornealen Lentikelextraktionen oder bei Keratoplastiken möglich.

Die Steueranordnung kann ferner vorzugsweise dazu eingerichtet sein, bei der Nachregulierung des Fokuspunkts in z-Richtung an mehreren verschiedenen Stellen in einer zur z-Richtung orthogonalen x-y-Ebene unterschiedliche Stellgrößen für das steuerbare optische Element zu erzeugen. Dadurch ist es möglich, beispielsweise unterschiedlich starke Auswirkungen der Temperaturveränderungen auf die Lage der Kontaktfläche in der x-y-Ebene individuell auszugleichen.

Die Messeinrichtung ist vorzugsweise eine kohärenzoptische interferometrische Messeinrichtung und besitzt hierzu ein optisches Interferometer.

Die Kontaktfläche wird vorzugsweise häufig Teil einer austauschbar angeordneten Wegwerfkomponente, beispielsweise eines Einweg-Applikators, sein. Es ist jedoch zu betonen, dass kein Wegwerfcharakter des die Kontaktfläche tragenden Elements voraussetzt wird. Vorzugsweise ist die Vorrichtung einsetzbar bei Ausgestaltungen mit fest eingebauter oder zumindest mehrfach verwendbarer Kontaktfläche.

Die Kontaktfläche ist bevorzugt von einer transparenten Applanationsplatte oder einem transparenten Kontaktglas gebildet. Applanationsplatten besitzen zumindest auf ihrer augenzugewandten Plattenseite eine ebene Applanationsfläche, mit der eine Einebnung der Augenvorderseite erzielt wird. Die Verwendung von Applanationsplatten zur Referenzierung des zu behandelnden Auges ist regelmäßig günstig unter dem Gesichtspunkt einer hohen Strahlqualität der Laserstrahlung. Vorzugsweise ist es möglich, als Kontaktelement ein Kontaktglas mit einer typischerweise konkav oder konvex geformten augenzugewandten Linsenfläche zu verwenden. Der Vorteil solcher Kontaktgläser ist z. B. ein geringerer Anstieg des Augeninnendrucks beim Andrücken an das Auge.

Die Kontaktfläche ist vorzugsweise von einem transparenten Kontaktelement gebildet, welches Teil eines mit einem Fokussierobjektiv der Vorrichtung insbesondere austauschbar gekoppelten Patientenadapters ist.

Es ist ferner ein Verfahren zur Steuerung eines Fokuspunkts eines Behandlungslaserstrahls für die ophthalmologische Laserchirurgie vorgesehen, umfassend die Schritte:
- Abbilden eines Behandlungslaserstrahls auf einen Fokuspunkt mittels eines Abbildungssystems,
- Messen einer dem Abbildungssystem zugeordneten Temperatur, und
- Steuern der Fokuspunkteinstellung abhängig von der gemessenen Temperatur.

Das Verfahren umfasst weiter die Schritte des Herstellens eines formenden Anlagekontakts zwischen einem Auge und einer Kontaktfläche und des Richten des Behandlungslaserstrahls durch die Kontaktfläche hindurch auf das Auge.

Auch bei dem Verfahrensaspekt werden Positionsmessdaten, welche für eine gemessene Position der Kontaktfläche an mindestens einer Stelle derselben bezogen auf die Ausbreitungsrichtung des Behandlungslaserstrahls repräsentativ sind, wie zuvor beschrieben ermittelt oder ausgelesen. Unabhängig davon, ob die Positionsmessdaten ermittelt oder ausgelesen wurden, wird der Fokuspunkt abhängig von den Positionsmessdaten voreingestellt und kann im Anschluss daran basierend auf der gemessenen Temperatur nachreguliert werden.

Die Erfindung wird nachfolgend anhand der beigefügten Zeichnungen weiter erläutert. Es zeigen:
- Fig. 1: stark schematisiert ein erstes Ausführungsbeispiel einer Vorrichtung für die ophthalmologische Laserchirurgie; und
- Fig. 2: stark schematisiert ein zweites Ausführungsbeispiel einer Vorrichtung für die ophthalmologische Laserchirurgie.

Die laserchirurgische Vorrichtung gemäß beider Ausführungsformen ist allgemein mit 10 bezeichnet.

Die laserchirurgische Vorrichtung 10 gemäß der ersten Ausführungsform weist einen Femtosekunden-Laser (Fs-Laser) 12 auf, welcher gepulste Laserstrahlung mit Pulsdauern im Bereich von Femtosekunden abgibt. Die Laserstrahlung breitet sich längs eines optischen Strahlengangs 14 aus und gelangt schließlich auf ein zu behandelndes Auge 16. In dem Strahlengang 14 sind verschiedene Komponenten zur Führung und Formung der Laserstrahlung angeordnet. Insbesondere umfassen diese Komponenten ein Fokussierobjektiv 18 (beispielsweise ein F-Theta-Objektiv) sowie einen dem Objektiv 18 vorgeschalteten Scanner 20, mittels dessen die von dem Laser 12 bereitgestellte Laserstrahlung in einer zum Strahlengang 14 orthogonalen Ebene (x-y-Ebene) ablenkbar ist. Ein eingezeichnetes Koordinatensystem veranschaulicht diese Ebene sowie eine durch die Richtung des Strahlengangs 14 definierte z-Achse. Der Scanner 20 ist beispielsweise in an sich bekannter Weise aus einem Paar galvanometrisch gesteuerter Ablenkspiegel aufgebaut, welche jeweils für die Strahlablenkung in Richtung einer der die x-y-Ebene aufspannenden Achsen verantwortlich sind. Eine zentrale Steuereinheit 22 steuert den Scanner 20 nach Maßgabe eines in einem Speicher 24 gespeicherten Steuerprogramms, welches ein in dem Auge 16 zu erzeugendes Schnittprofil (repräsentiert durch ein dreidimensionales Muster von Abtastpunkten, an denen jeweils eine Photodisruption zu bewirken ist) implementiert.

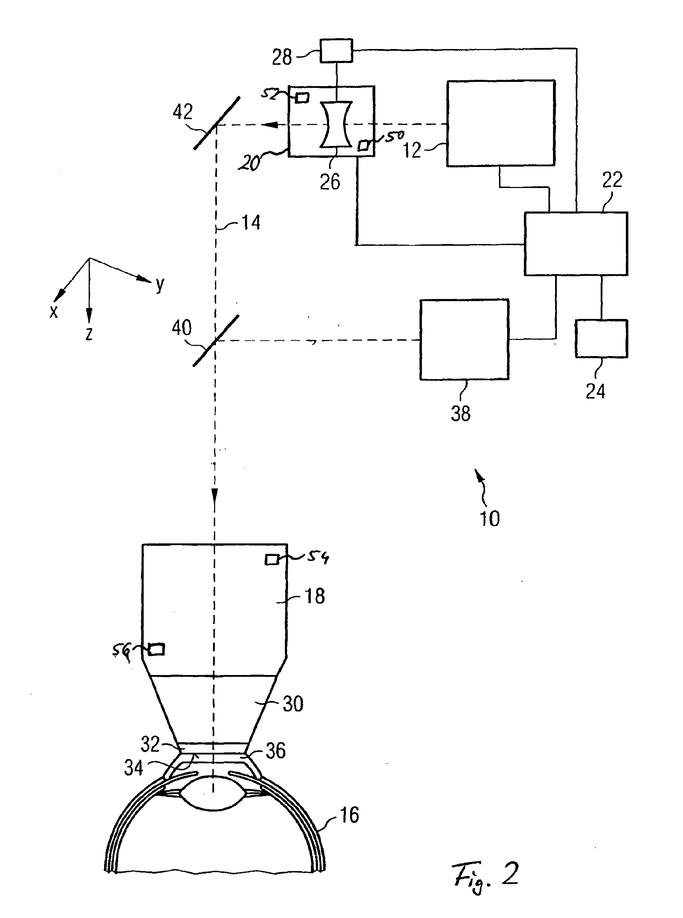

Des weiteren beinhalten die erwähnten Komponenten zur Führung und Formung der Laserstrahlung mindestens ein steuerbares optisches Element zur z-Verstellung des Strahlfokus der Laserstrahlung. Im gezeigten Beispielfall ist dieses optische Element von einer Linse gebildet. Zur Steuerung der Linse 26 dient ein geeigneter Aktuator 28, der seinerseits durch die Steuereinheit 22 gesteuert ist. Beispielsweise kann die Linse 26 mechanisch längs des optischen Strahlengangs 14 verfahrbar sein. Alternativ ist es vorstellbar, eine steuerbare Flüssiglinse variabler Brechkraft zu verwenden. Bei unveränderter z-Position und auch ansonsten unveränderter Einstellung des Fokussierobjektivs 18 lässt sich durch Verfahren einer längsverstellbaren Linse oder durch Brechkraftvariation einer Flüssiglinse eine z-Verlagerung des Strahlfokus erreichen. Es versteht sich, dass zur z-Verstellung des Strahlfokus auch andere Komponenten vorstellbar sind, etwa ein verformbarer Spiegel. Wegen seiner vergleichsweise höheren Trägheit ist es zweckmäßig, mit dem Fokussierobjektiv 18 nur eine anfängliche Grundeinstellung des Strahlfokus (d.h. Fokussierung auf eine vorgegebene z-Referenzposition) vorzunehmen, die durch das Schnittprofil vorgegebenen z-Verlagerungen des Strahlfokus jedoch durch eine außerhalb des Fokussierobjektivs 18 angeordnete Komponente mit kürzerer Reaktionsgeschwindigkeit zu bewerkstelligen. Es versteht sich, dass die Linse 26 auch Teil des Scanners 20 sein kann und der dadurch gebildete Scanner 20 sowohl vor als auch nach dem halbdurchlässigen Umlenkspiegel 40 angeordnet sein kann. Der Fall, bei welchem die Linse Teil des Scanners 20 ist und dieser die Linse 26 enthaltende Scanner 20 vor dem Umlenkspiegel 42 angeordnet ist, wird später in Bezug auf Figur 2 erläutert.

Auf der Seite des Strahlaustritts ist das Fokussierobjektiv 18 mit einem Patientenadapter 30 gekoppelt, welcher zur Herstellung einer mechanischen Kopplung zwischen dem Auge 16 und dem Fokussierobjektiv 18 dient. Üblicherweise wird bei Behandlungen der hier betrachteten Art ein in der Zeichnung nicht näher dargestellter, für sich jedoch bekannter Saugring auf das Auge aufgesetzt und dort durch Saugkraft fixiert. Der Saugring und der Patientenadapter 30 bilden eine definierte mechanische Schnittstelle, welche eine Ankopplung des Patientenadapters 30 an den Saugring gestattet. Diesbezüglich kann beispielsweise auf die internationale Patentanmeldung PCT/EP2008/006962 verwiesen werden, deren Gesamtinhalt hiermit durch Verweis einbezogen wird.

Der Patientenadapter 30 dient als Träger für ein transparentes Kontaktelement, welches im gezeigten Beispielfall als planparallele Applanationsplatte ausgebildet ist. Der Patientenadapter 30 umfasst beispielsweise einen Kegelhülsenkörper, an dessen schmälerem (in der Zeichnung unterem) Hülsenende die Applanationsplatte 32 angeordnet ist. Im Bereich des breiteren (in der Zeichnung oberen) Hülsenendes ist der Patientenadapter 30 dagegen an das Fokussierobjektiv 18 angesetzt und besitzt dort geeignete Formationen, die eine gewünschtenfalls lösbare Fixierung des Patientenadapters 30 an dem Fokussierobjektiv 18 gestatten.

Weil sie während der Behandlung in Kontakt mit dem Auge 16 gelangt, ist die Applanationsplatte 32 ein unter dem Gesichtspunkt der Hygiene kritischer Artikel, der deshalb zweckmäßigerweise nach jeder Behandlung auszuwechseln ist. Hierzu kann die Applanationsplatte 32 auswechselbar an dem Patientenadapter 30 angebracht sein. Alternativ kann der Patientenadapter 30 zusammen mit der Applanationsplatte 32 eine Wegwerfeinheit bilden, wozu die Applanationsplatte 32 unlösbar mit dem Patientenadapter 30 verbunden sein kann.

Jedenfalls bildet die augenzugewandte Unterseite der Applanationsplatte 32 eine ebene Kontaktfläche 34, gegen welche zur Vorbereitung der Behandlung das Auge 16 gedrückt wird. Dies bewirkt eine Einebnung der Augenvorderfläche unter gleichzeitiger Verformung der mit 36 bezeichneten Kornea des Auges 16.

Um die Kontaktfläche 34 als Referenz für die Voreinstellung des Strahlfokus in z-Richtung nutzen zu können, ist es nötig, ihre z-Lage in dem Koordinatensystem der laserchirurgischen Vorrichtung zu kennen. Aufgrund unvermeidlicher Fertigungstoleranzen kann nicht ausgeschlossen werden, dass bei Einbau unterschiedlicher Applanationsplatten bzw. unterschiedlicher Patientenadapter 30, die jeweils mit einer Applanationsplatte 32 bestückt sind, die z-Lage und unter Umständen auch die Winkellage der Kontaktfläche 34 mehr oder weniger signifikante Schwankungen zeigt. Soweit diese Schwankungen bei der z-Voreinstellung des Strahlfokus unberücksichtigt bleiben, ergeben sich unerwünschte Fehler bei der tatsächlichen Lage der erzeugten Inzisionen im Auge 16.

Deshalb enthält die laserchirurgische Vorrichtung 10 eine kohärenzoptische interferometrische Messeinrichtung 38, beispielsweise eine OLCR-Messeinrichtung (OLCR: Optical Low Coherence Reflectrometry), welche einen Messstrahl aussendet, der mittels eines unbeweglich angeordneten, halbdurchlässigen Umlenkspiegels 40 in den Strahlengang 14 eingekoppelt wird, in dem auch die Behandlungs-Laserstrahlung des Lasers 12 läuft. Die Messeinrichtung 38 bringt den erzeugten Messstrahl mit einem vom Auge 16 zurückkommenden Reflektionsstrahl in Interferenz. Aus den diesbezüglich gewonnenen Interferenzmessdaten kann die z-Position der Kontaktfläche 34 innerhalb des Koordinatensystems der laserchirurgischen Vorrichtung ermittelt werden. Deshalb kann man die Interferenzmessdaten auch als Positionsmessdaten bezeichnen. Die Steuereinheit 22 erhält die Interferenzmessdaten von der Messeinrichtung 38 und berechnet hieraus die z-Lage derjenigen Stelle der Kontaktfläche 34, an welcher der Messstrahl auftraf bzw. durch welche der Messstrahl hindurchging.

Im gezeigten Beispielfall durchläuft der von der Messeinrichtung 38 ausgesendete Messstrahl den Scanner 20. Dies ermöglicht es, die Ablenkfunktion des Scanners 20 auch für den Messstrahl zu nutzen. Das Scannermodul 20 könnte auch einen zweiten separaten Scanner allein für das OLCR enthalten, der mit kleineren Spiegeln ausgestattet deutlich schneller arbeitet.

Bei der folgenden Laserbehandlung des Auges 16 berücksichtigt die Steuereinheit 22 die so ermittelte tatsächliche z-Lage der Kontaktfläche 34 bei der z-Steuerung des Strahlfokus, und zwar so, dass die Inzision tatsächlich an der beabsichtigten Position in der Tiefe der Kornea 36 erzeugt wird. Hierzu referenziert die Auswerte- und Steuereinheit 22 die einzustellende z-Position des Strahlfokus auf die gemessene z-Position der Kontaktfläche 34.

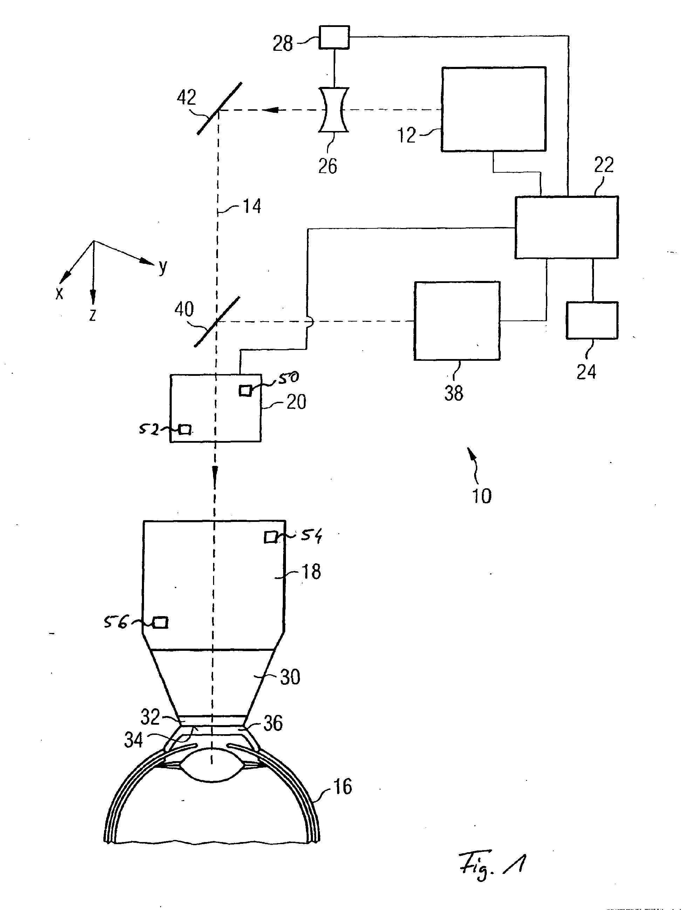

Durch die zuvor beschriebene Vorgehensweise wird die z-Position des Strahlfokus jedoch nur voreingestellt, da Temperaturdrifte der effektiven Brennweite der laserchirurgischen Vorrichtung 10 sowie der realen optischen Länge des Patientenadapters 30 in z-Richtung nicht berücksichtigt werden. Dementsprechend weist die laserchirurgische Vorrichtung 10 vier Temperatursensoren 50, 52, 54, 56 auf, von denen zwei an dem Scanner 20 und zwei an dem Fokussierobjektiv 18 angeordnet sind. Die Temperatursensoren messen an ihren entsprechenden Positionen die reale Temperatur und geben die gemessenen Temperaturwerte an die Steuereinheit 22 weiter. Die Weitergabe der Temperaturwerte an die Steuereinheit 22 kann drahtlos oder drahtgebunden erfolgen, d.h. die Temperatursensoren 50, 52, 54, 56 können drahtlos oder drahtgebunden mit der Steuereinheit 22 in Verbindung stehen. In dem in der Figur 1 dargestellten Ausführungsbeispiel sind beispielhaft der Scanner 20 und damit die an dem Scanner 20 angeordneten Temperatursensoren 50, 52 mit der Steuereinheit 22 drahtgebunden verbunden, während die an dem Fokussierobjektiv 18 angeordneten Temperatursensoren 54, 56 drahtlos mit der Steuereinheit 22 verbunden sind, um ihre Temperaturmesswerte an die Steuereinheit 22 zur weiteren Verarbeitung weiterzugeben.

In dem Speicher 24 ist sowohl für den Scanner 20 als auch für das Fokussierobjektiv 18 ein Temperaturgang der effektiven Brennweite als Kurvenschar hinterlegt. Die Steuereinheit wertet bei Vorliegen eines neuen Temperaturmesswerts die zugehörige Funktion aus und erzeugt eine entsprechende Stellgröße zur Nachregulierung der voreingestellten z-Position der Linse 26. Wird durch einen oder beide der an dem Scanner 20 angebrachten Temperatursensoren 50, 52 ein Temperaturwert ermittelt (bei der Messung von zwei Temperaturwerten durch die beiden Temperatursensoren 50, 52 wird ein aus den beiden Werten gebildeter Durchschnittstemperaturwert verwendet), gibt der Temperatursensor den gemessenen Temperaturwert an die Steuereinheit 22 weiter. Diese durchsucht dann den Speicher 20 nach dem zugehörigen Temperaturgang für den Scanner 20, erzeugt daraus eine Stellgröße und übermittelt diese an den Aktuator 28, der die Linse 26 entsprechend der Stellgröße in z-Richtung verschiebt. Durch diese z-Verschiebung der Linse 26 wird die voreingestellte Position des Strahlfokus derart nachreguliert, dass auch auf Grund von Schwankungen der realen Temperatur auftretende Veränderungen der realen optischen Länge des Patientenadapters und/oder der effektiven Brennweite der laserchirurgischen Vorrichtung 10 berücksichtigt und ausgeglichen werden.

Gemäß der in Figur 2 gezeigten zweiten Ausführungsform der laserchirurgischen Vorrichtung 10 umfasst der Scanner 20 die in Ausbreitungsrichtung des Behandlungslaserstrahls positionsverschiebbare Linse 26 und ist in Ausbreitungsrichtung der Laserstrahlung vor dem Umlenkspiegel 42 angeordnet. Auf diese Weise ist der Scanner 20 ein 3D-Scanner, der dreidimensionale Scaneigenschaften besitzt, so dass die Laserstrahlung in jede Richtung (x, y, z) von dem 3D-Scanner 20 abgelenkt werden kann.

Die Aufnahme und Auswertung der Temperaturmesswerte mittels der Temperatursensoren 50, 52, 54, 56 und der Steuereinheit 22 erfolgt analog der in Figur 1 gezeigten ersten Ausführungsform. Im Unterschied zu der ersten Ausführungsform erfolgt in der in Figur 2 gezeigten zweiten Ausführungsform sowohl die FokuspunktVoreinstellung als auch die Fokuspunkt-Nachregulierung durch den 3D-Scanner 20, der durch die Steuereinheit 22 gesteuert wird.

## Patentansprüche

1. Vorrichtung (10) für die ophthalmologische Laserchirurgie, umfassend
- ein optisches Abbildungssystem, das eingerichtet ist, um einen Behandlungslaserstrahl (14) auf einen Fokuspunkt abzubilden,
- eine Temperaturmesseinrichtung, die eingerichtet ist, um eine dem Abbildungssystem zugeordnete Temperatur zu messen,
- eine mit der Temperaturmesseinrichtung verbundene elektronische Steueranordnung (22), welche dazu eingerichtet ist, die Fokuspunkteinstellung abhängig von der gemessenen Temperatur zu steuern,
- eine Kontaktfläche (34), die eingerichtet ist, um eine Anlage eines zu behandelnden Auges (16) zu formen, und
- eine Messeinrichtung (38), die eingerichtet ist, um eine Positionsvermessung der Kontaktfläche (34) bezogen auf die Ausbreitungsrichtung des Behandlungslaserstrahls (14) durchzuführen, wobei die Messeinrichtung (38) eingerichtet ist, um Positionsmessdaten bereitzustellen, welche für die gemessene Position der Kontaktfläche (34) an mindestens einer Stelle derselben repräsentativ sind, wobei die elektronische Steueranordnung (22) dazu eingerichtet ist, den Fokuspunkt abhängig von den Positionsmessdaten voreinzustellen.

2. Vorrichtung (10) nach Anspruch 1, wobei die Temperaturmesseinrichtung einen oder mehrere Temperatursensoren (50, 52, 54, 56) umfasst, die an zumindest einer von optischen Komponenten (18, 20, 42) des optischen Abbildungssystems angeordnet und mit der elektronischen Steueranordnung (22) verbunden sind.

3. Vorrichtung (10) nach Anspruch 1 oder 2, wobei die optischen Komponenten (18, 20, 42) zumindest ein steuerbares optisches Element (26) zur Steuerung des Fokuspunkts aufweisen.

4. Vorrichtung (10) nach Anspruch 3, wobei das steuerbare optische Element (26) zumindest eine in Ausbreitungsrichtung des Behandlungslaserstrahls (14) positionsveränderbare Linse aufweist.

5. Vorrichtung (10) nach Anspruch 4, wobei die Steueranordnung (22) dazu eingerichtet ist, zur Steuerung des Fokuspunkt eine Stellgröße zur Veränderung der Position der positionsveränderbaren Linse zu erzeugen.

6. Vorrichtung (10) nach einem der Ansprüche 3 bis 5, wobei die Steueranordnung (22) eine Speichereinheit (24) aufweist, in welcher die Abhängigkeit des Fokuspunkts von der Temperatur als eine Funktion gespeichert ist und die Steueranordnung (22) dazu eingerichtet ist, den Fokuspunkt basierend auf der gespeicherten Funktion und der gemessenen Temperatur zu steuern.

7. Vorrichtung (10) nach einem der Ansprüche 1 bis 6, wobei die Messeinrichtung (38) eine einen Messstrahl bereitstellende Strahlungsquelle umfasst und die optischen Komponenten (18, 20, 42) dazu ausgebildet und angeordnet sind, auch den Messstrahl durch die Kontaktfläche hindurch auf das Auge zu richten.

8. Vorrichtung (10) nach einem der Ansprüche 1 bis 7, wobei die Messeinrichtung (38) ein optisches Interferometer umfasst.

9. Vorrichtung (10) nach einem der Ansprüche 1 bis 8, wobei die Kontaktfläche (34) Teil einer austauschbar angeordneten Wegwerfkomponente ist.

10. Vorrichtung (10) nach einem der vorhergehenden Ansprüche, wobei die Pulsdauer des Behandlungslaserstrahls (14) im Femtosekundenbereich liegt.

11. Verfahren zur Steuerung eines Fokuspunkts eines Behandlungslaserstrahls für die ophthalmologische Laserchirurgie, umfassend die Schritte:
- Abbilden eines Behandlungslaserstrahls (14) auf einen Fokuspunkt mittels eines Abbildungssystems,
- Messen einer dem Abbildungssystem zugeordneten Temperatur,
- Steuern der Fokuspunkteinstellung abhängig von der gemessenen Temperatur,
- Herstellen eines formenden Anlagekontakts zwischen einem Auge (16) und einer Kontaktfläche (34),
- Richten des Behandlungslaserstrahls (14) durch die Kontaktfläche (34) hindurch auf das Auge (16),
- Erzeugen von Positionsmessdaten, welche für eine gemessene Position der Kontaktfläche (34) an mindestens einer Stelle derselben bezogen auf die Ausbreitungsrichtung des Behandlungslaserstrahls (14) repräsentativ sind, und
- Voreinstellen des Fokuspunkts abhängig von den Positionsmessdaten.

## Claims

1. Apparatus (10) for ophthalmological laser surgery, comprising
- an optical imaging system adapted to image a treatment laser beam (14) onto a focal point,
- a temperature measuring device adapted to measure a temperature assigned to the imaging system,
- an electronic control arrangement (22) connected to the temperature measuring device, which is adapted to control the focal-point setting depending on the measured temperature,
- a contact surface (34) adapted to shape an abutment of an eye (16) to be treated, and
- a measuring device (38) adapted to perform a positional surveying of the contact surface (34) relative to the direction of propagation of the treatment laser beam (14), the measuring device (38) being adapted to provide measured position data that are representative of the measured position of the contact surface (34) at at least one place on the same, the electronic control arrangement (22) being adapted to preset the focal point depending on the measured position data.

2. Apparatus (10) according to claim 1, wherein the temperature measuring device comprises one or more temperature sensors (50, 52, 54, 56), which are arranged on at least one of optical components (18, 20, 42) of the optical imaging system and being connected to the electronic control arrangement (22).

3. Apparatus (10) according to claim 1 or 2, wherein the optical components (18, 20, 42) have at least one controllable optical element (26) for controlling the focal point.

4. Apparatus (10) according to claim 3, wherein the controllable optical element (26) has at least one lens that is positionally variable in the direction of propagation of the treatment laser beam (14).

5. Apparatus (10) according to claim 4, wherein the control arrangement (22) is adapted to generate, for the purpose of controlling the focal point, an actuating variable for varying the position of the positionally variable lens.

6. Apparatus (10) according to any one of claims 3 to 5, wherein the control arrangement (22) has a memory unit (24), in which the dependence of a focal point on the temperature is stored as a function, and the control arrangement (22) is adapted to control the focal point based on the stored function and the measured temperature.

7. Apparatus (10) according to any one of claims 1 to 6, wherein the measuring device (38) comprises a radiation source providing a measuring beam, and wherein the optical components (18, 20, 42) are designed and arranged to direct also the measuring beam through the contact surface onto the eye.

8. Apparatus (10) according to any one of claims 1 to 7, wherein the measuring device (38) comprises an optical interferometer.

9. Apparatus (10) according to any one of claims 1 to 8, wherein the contact surface (34) is part of an exchangeably arranged disposable component.

10. Apparatus (10) according to any one of the preceding claims, wherein the pulse duration of the treatment laser beam (14) lies within the femtosecond range.

11. Method for controlling a focal point of a treatment laser beam for ophthalmological laser surgery, comprising the following steps:
- imaging a treatment laser beam (14) onto a focal point by means of an imaging system,
- measuring a temperature assigned to the imaging system,
- controlling the focal point setting depending on the measured temperature,
- establishing a shaping abutment between an eye (16) and a contact surface (34),
- directing the treatment laser beam (14) through the contact surface (34) onto the eye (16),
- generating measured position data that are representative of a measured position of the contact surface (34) at at least one place on the same, relative to the direction of propagation of the treatment laser beam (14), and
- presetting the focal point depending on the measured position data.

## Revendications

1. Dispositif (10) pour la chirurgie ophtalmologique par laser, comprenant
- un système d'imagerie optique agencé de manière à représenter un faisceau laser de traitement (14) sur un point focal,
- un dispositif de mesure de température agencé de manière à mesurer une température associée audit système d'imagerie,
- un dispositif de commande électronique (22) connecté audit dispositif de mesure de température et agencé de manière à commander le réglage du point focal en fonction de la température mesurée,
- une surface de contact (34) agencée de manière à former un appui pour l'oeil (16) à traiter, et
- un dispositif de mesure (38) agencé de manière à mesurer la position de ladite surface de contact (34) par rapport à la direction de propagation du faisceau laser de traitement (14), ledit dispositif de mesure (38) étant agencé de manière à fournir des données de mesure de position, lesquelles sont représentatives de la position de la surface de contact (34), mesurée au moins en un point de celle-ci, le dispositif de commande électronique (22) étant agencé de manière à prérégler le point focal en fonction des données de mesure de position.

2. Dispositif (10) selon la revendication 1, le dispositif de mesure de température comportant un ou plusieurs capteurs de température (50, 52, 54, 56) qui sont disposés sur au moins un des composant optiques (18, 20, 42) du système d'imagerie optique et connectés au dispositif de commande électronique (22).

3. Dispositif (10) selon la revendication 1 ou 2, lesdits composants optiques (18, 20, 42) présentant au moins un élément optique (26) pouvant être commandé pour la commande du point focal.

4. Dispositif (10) selon la revendication 3, ledit élément optique (26) commandable présentant au moins une lentille positionnable de manière variable dans la direction de propagation du faisceau laser de traitement (14).

5. Dispositif (10) selon la revendication 4, le dispositif de commande électronique (22) étant agencé de manière à générer, en vue de la commande du point focal, une grandeur de réglage pour la modification de la position de la lentille positionnable de manière variable.

6. Dispositif (10) selon l'une des revendications 3 à 5, le dispositif de commande électronique (22) présentant une unité de mémoire (24) dans laquelle la dépendance du point focal à l'égard de la température est enregistrée sous la forme d'une fonction, et le dispositif de commande électronique (22) étant agencé de manière à commander le point focal sur la base de ladite fonction enregistrée et de la température mesurée.

7. Dispositif (10) selon l'une des revendications 1 à 6, le dispositif de mesure (38) comprenant une source de rayonnement fournissant un faisceau de mesure, et les composants optiques (18, 20, 42) étant conçus et disposés de manière à diriger le faisceau de mesure sur l'oeil, à travers la surface de contact.

8. Dispositif (10) selon l'une des revendications 1 à 7, le dispositif de mesure (38) comprenant un interféromètre optique.

9. Dispositif (10) selon l'une des revendications 1 à 8, la surface de contact (34) faisant partie intégrante d'un composant jetable disposé de façon à pouvoir être remplacé.

10. Dispositif (10) selon l'une des revendications précédentes, la durée d'impulsion du faisceau laser de traitement (14) étant de l'ordre des femtosecondes.

11. Procédé pour la commande d'un point focal d'un faisceau laser de traitement pour la chirurgie ophtalmologique par laser, comprenant les étapes suivantes :
- la représentation visuelle d'un faisceau laser de traitement (14) sur un point focal à l'aide d'un système d'imagerie,
- la mesure d'une température associée au système d'imagerie,
- la commande du réglage du point focal en fonction de la température mesurée,
- l'établissement d'un contact formant appui entre un oeil (16) et une surface de contact (34),
- l'orientation du faisceau laser de traitement (14) sur l'oeil (16) à travers la surface de contact (34),
- la genèse de données de mesure de position, lesquelles sont représentatives d'une position de la surface de contact (34),mesurée au moins en un point de celle-ci par rapport à la direction de propagation du faisceau laser de traitement (14), et
- le préréglage du point focal en fonction des données de mesure de position.
